# EUROPEAN PATENT APPLICATION

(11) **EP 4 775 540 A1**
(43) Date of publication of application: **15.07.2026**
(21) Application number: 24862915.6
(22) Date of filing: 06.09.2024
(51) Int. Cl.: C01G 23/04, B01J 27/053, B01J 35/61, B01J 37/04, C07D 307/48

(54) **METHOD FOR PRODUCING 5-HYDROXYMETHYLFURFURAL**

(30) Priority: 08.09.2023 JP 2023146434
(71) Applicant: Green Chemical Inc., Fujisawa-shi, Kanagawa 251-8555 (JP)
(72) Inventor: CHO, Kinryo, Fujisawa-shi, Kanagawa 251-8555 (JP); IWAKOSHI, Banri, Fujisawa-shi, Kanagawa 251-8555 (JP); ITOU, Satoshi, Fujisawa-shi, Kanagawa 251-8555 (JP)
(74) Representative: IK-IP LTD
(86) International application number: PCT/JP2024/032072
(87) International publication number: WO 2025/053266

(57) **Abstract**

An object of the present invention is to provide conditions suitable for industrial production in a method for producing 5-hydroxymethylfurfural from an aqueous sugar solution such as glucose.

[Solution]

The method includes heating an aqueous sugar solution containing dimethyl sulfoxide and/or a polar protic organic solvent in the presence of a compound having a sulfonic acid group covalently bonded to a titanium oxide, or the like, or heating an aqueous saccharide solution by microwave irradiating in the presence of a compound having a sulfonic acid group covalently bonded to a titanium oxide, or the like.

## Description

### [Technical Field]

The present invention relates to a compound that is useful as a catalyst for converting saccharides such as glucose or fructose into 5-hydroxymethylfurfural (hereinafter sometimes referred to as "HMF" herein). Furthermore, the present invention relates to a method for producing HMF from an aqueous solution of saccharides such as glucose or fructose using the aforementioned compound as a catalyst.

### [Background Art]

HMF is an important chemical intermediate that can be synthesized from glucose as a starting material; however, the synthetic pathway thereof is very complex. Glucose as the starting material is converted into fructose by skeletal isomerization in the presence of an acid catalyst. The resulting fructose is converted into HMF by a dehydration reaction in the presence of an acid catalyst; however, HMF is further hydrolyzed sequentially by the acid catalyst in the reaction system to form organic acids, such as formic acid and levulinic acid. Therefore, there has been a demand for a novel catalyst capable of producing HMF with high selectivity.

A solid Lewis acid comprising a phosphorylated titanium oxide, in which phosphate residues are covalently bonded to the surface of an amorphous hydrated titanium oxide obtained by treating the amorphous hydrated titanium oxide with phosphoric acid, is known as a catalyst for converting glucose or fructose into HMF (see Patent Document 1). Specifically, 0.05 g of the above-mentioned phosphorylated titanium oxide is added to a glucose solution containing 0.02 g (0.11 mmol) of D-glucose in 0.2 mL of distilled water and 1.8 mL of tetrahydrofuran (THF), the mixture is allowed to react at 20°C for 2 hours, thereby obtaining HMF at a conversion rate of 98% and a yield of 81.2%.

### [Prior Art Documents]

### [Patent Documents]

Patent Document 1 : International Publication No. 2012-108472

### [Summary of the Invention]

### [Problem to be Solved by the Invention]

However, the use of a large amount of THF as a solvent is industrially problematic, and it has been necessary to reduce the amount of organic solvent used as much as possible. An object of the present invention is to provide, in a method for producing HMF from saccharides such as glucose or fructose, conditions that enable industrial production.

### [Means for Solving the Problem]

The present inventors, as a result of intensive studies to solve the above-described problem, have found a novel catalyst capable of effectively converting saccharides such as glucose or fructose and have found that the above-described problem can be solved by using, as a reaction solvent for a reaction of converting saccharides such as glucose or fructose into HMF, a mixed solvent of water and dimethyl sulfoxide and/or a polar protic organic solvent, and further by carrying out the reaction of converting saccharides such as glucose or fructose into HMF under microwave irradiation, thereby having completed the present invention.

That is, the present invention relates to a compound having a sulfonic acid group covalently bonded to a titanium oxide. The BET specific surface area of the compound is preferably 200 m²/g or more. The BET specific surface area of the compound is preferably 300 m²/g or less. The compound is preferably a solid Lewis acid. The compound is preferably capable of catalyzing a chemical reaction in an aqueous solution. The chemical reaction is preferably the dehydration reaction of saccharides to produce 5-hydroxymethylfurfural from saccharides. Furthermore, the saccharides are preferably glucose and/or fructose. The present invention also relates to a method for producing 5-hydroxymethylfurfural, comprising a step of heating an aqueous saccharide solution in the presence of the compound. The present invention also relates to a method A for producing 5-hydroxymethylfurfural, comprising a step of heating an aqueous saccharide solution A containing dimethyl sulfoxide (sometimes referred to herein as "DMSO" herein) and/or a polar protic organic solvent in the presence of the compound or a compound having a phosphono group covalently bonded to a titanium oxide.
The present invention also relates to a method B for producing 5-hydroxymethylfurfural, comprising a step of heating a saccharide aqueous solution B by irradiating with microwaves in the presence of the compound or a compound having a phosphono group covalently bonded to a titanium oxide. The saccharide aqueous solution B is preferably an aqueous solution containing dimethyl sulfoxide and/or a polar protic organic solvent. The polar protic organic solvent is preferably an alcohol having 1 to 6 carbon atoms, and more preferably an alcohol having 3 to 5 carbon atoms. The saccharides are preferably glucose and/or fructose. The compound having a phosphono group covalently bonded to a titanium oxide is preferably a solid Lewis acid.
The present invention also relates to a method for producing the aforementioned compound, comprising treating a titanium oxide with sulfuric acid.

### [Effects of the Invention]

The compound and the production method of the present invention are suitable for industrial production of HMF because the amount of organic solvent used can be significantly reduced.

### [Modes for Carrying Out the Invention]

The compound of the present invention is a compound in which a sulfonic acid group (-SO₃H) is covalently bonded to a titanium oxide. The titanium oxide is not particularly limited, but examples include crystalline titanium oxides such as rutile-type, anatase-type, and brookite-type titanium oxides; amorphous titanium oxides; hydrated titanium oxides such as orthotitanic acid and metatitanic acid; titanium hydroxide; composite oxides of titanium and other metals such as lithium titanate, barium titanate, and strontium titanate; and hydrolysis products of titanium oxide precursors. These may be used alone or in combination of two or more thereof.

The titanium oxide may be doped with a metal element (excluding titanium; the same applies hereinafter), and may be coated with a compound containing a metal element. Examples of the metal element include aluminum, gallium, indium, thallium, magnesium, manganese, calcium, niobium, tantalum, zirconium, silicon, zinc, cerium, iron, tungsten, molybdenum, and vanadium. When the titanium oxide is doped with a metal element, the doping ratio of the metal element relative to the number of moles of titanium atoms is preferably 10 mol% or less, more preferably 5 mol% or less, even more preferably 1 mol% or less, and particularly preferably 0.5 mol% or less.

The titanium oxide can be obtained, for example, by hydrolyzing a titanium oxide precursor. Examples of the titanium oxide precursor include inorganic titanium compounds such as titanium hydroxide, titanic acid, titanium trichloride, titanium tetrachloride, titanium tetrabromide, titanium sulfate, and titanium oxysulfate; titanium alkoxide compounds such as titanium tetraisopropoxide, titanium tetra-n-butoxide, tetrakis(2-ethylhexyloxy)titanium, and titanium tetrastearyloxide; titanium acylate compounds; titanium chelate compounds such as titanium(IV) bis(acetylacetonate) diisopropoxide, isopropoxy(2-ethyl-1,3-hexanediolato)titanium, and titanium bis(lactato)hydroxide; organic acid titanium compounds such as titanium oxalate and titanium tetraacetate; and water-soluble titanium complexes. Among these, the titanium oxide precursor is preferably at least one selected from the group consisting of titanium chlorides, titanium oxalates, titanium sulfates, and titanium alkoxides.

The number of carbon atoms constituting the alkoxy group in the titanium alkoxide is preferably 1 to 6, and more preferably 2 to 4. The alkoxy group may be linear, branched, or cyclic.

The hydrolysis reaction of the titanium oxide precursor can be carried out by reacting the titanium oxide precursor with water, optionally in the presence of an acid catalyst or a base catalyst. The temperature during the hydrolysis reaction is preferably 10 to 100 °C, and more preferably 20 to 50 °C. The time required for the hydrolysis reaction can be appropriately set depending on factors such as the reaction temperature and reaction scale, but is preferably 0.5 hours to 48 hours, and more preferably 10 hours to 24 hours.

The hydrolysis reaction can be performed repeatedly a plurality of times. Examples of the acid used in the hydrolysis reaction include hydrochloric acid, sulfuric acid, and nitric acid. Examples of the base used in the hydrolysis reaction include ammonia, sodium hydroxide, and potassium hydroxide.

The mixing ratio of the titanium oxide precursor to the acid catalyst or the base catalyst is not particularly limited, but is usually preferably in the range of a molar ratio of 1:0.1 to 1:10, and more preferably in the range of 1:1 to 1:5. In addition, in the hydrolysis reaction, the initial concentration of the titanium oxide precursor in the total reaction solution including water is preferably in the range of 1 to 50 mass %, and more preferably in the range of 10 to 20 mass %.

The hydrolysis product of the titanium oxide precursor is preferably an amorphous hydrated titanium oxide. Whether the amorphous hydrated titanium oxide contains water molecules can be confirmed by surface measurement using an infrared spectroscopy or by weight loss due to heat dehydration treatment under vacuum. In addition, whether it is amorphous can be examined by X-ray diffraction analysis.

In the present invention, the term "amorphous" means that the material contains an amorphous portion that can be identified by X-ray diffraction and includes not only cases where the entire amorphous hydrated titanium oxide is amorphous, but also cases where only a part thereof is amorphous. The amorphous pattern identifiable by X-ray diffraction specifically refers to a broad halo pattern in which a distinct crystalline diffraction pattern is absent.

The compound of the present invention is a compound in which a sulfonic acid group is covalently bonded to the titanium oxide. The compound can be obtained, for example, by treating the titanium oxide with sulfuric acid. Specifically, the sulfuric acid treatment can be carried out by immersing the titanium oxide in an aqueous sulfuric acid solution (for example, a 0.5-2 M sulfuric acid aqueous solution) and stirring the resulting mixture.

The temperature for the sulfuric acid treatment is preferably in the range of 10°C to 100°C, and more preferably in the range of 20°C to 40°C. The duration of the sulfuric acid treatment is a period during which the desired compound to be produced and may be appropriately selected depending on conditions such as the treatment temperature. The duration is preferably in the range of 1 to 96 hours, and more preferably in the range of 24 to 72 hours.

The sulfuric acid-treated titanium oxide may be subjected to heat drying as necessary. Although the heating temperature is not particularly limited, a temperature sufficient to remove adhered water is preferred, and a temperature of 100°C or more is desirable. When vacuum heat drying is performed, the temperature may be appropriately adjusted depending on the degree of vacuum.

In the FT-IR spectrum, while the titanium oxide that has not been subjected to sulfuric acid treatment shows no peak in the vicinity of 1180 cm⁻¹, the solid Lewis acid of the present invention exhibits a signal (peak) attributable to the stretching vibration of the S-O bond in the vicinity of 1180 cm⁻¹. This indicates that a sulfonic acid group is covalently bonded to the hydroxyl group (Ti-OH) on the skeletal surface of the titanium oxide, forming a chemical structure of Ti-O-SO₂(OH). It is also preferred that the compound functions as a solid Lewis acid.

The compound of the present invention may also be bonded to functional groups other than sulfonic acid groups. However, among the functional groups covalently bonded to the titanium oxide, it is preferable that the sulfonic acid group has the largest mole fraction. Among the total number of functional groups covalently bonded to the titanium oxide, the proportion of sulfonic acid groups is preferably 60 mol% or more, more preferably 80 mol% or more, even more preferably 95 mol% or more, and particularly preferably 99 mol% or more. In cases where the titanium oxide is bonded to both sulfonic acid groups and phosphono groups, such titanium oxide is treated as the compound of the present invention, and not as a phosphorylated titanium oxide.

The titanium oxide in the compound may be doped with a metal element, and it may be coated with a compound containing a metal element. Examples of the metal element include aluminum, gallium, indium, thallium, magnesium, manganese, calcium, niobium, tantalum, zirconium, silicon, zinc, cerium, iron, tungsten, molybdenum, vanadium, and the like. When the metal element is doped into the titanium oxide, the doping ratio of the metal element relative to the number of moles of the titanium atoms is preferably 10 mol% or less, more preferably 5 mol% or less, and even more preferably 1 mol% or less.

The BET specific surface area of the compound of the present invention is not particularly limited, but in order to carry out the chemical reaction described later more efficiently, it is preferably 100 m²/g or more, and more preferably 200 m²/g or more. The inventors presume that setting the BET specific surface area to 100 m²/g or more sufficiently increases the number of active sites capable of contacting the target substances that promote the chemical reaction described later (for example, sugars such as glucose or fructose).

The BET specific surface area of the compound of the present invention is preferably 400 m²/g or less, and more preferably 300 m²/g or less, from the standpoint of achieving superior durability (stability of performance) when the catalyst is used in a chemical reaction described later. The inventors presume that by setting the BET specific surface area to 400 m²/g or less, the pore size of the catalyst of the present invention is maintained within an appropriate range, allowing substances generated or introduced inside the pores to exit the pores, thereby making it easier to maintain catalytic activity.

The BET specific surface area was determined by adsorbing nitrogen gas onto the surface of the target material (for example, the compound of the present invention) at liquid-nitrogen temperature, and obtaining an adsorption-desorption isotherm based on the adsorption and desorption of nitrogen gas at -196 °C. Prior to measurement, the sample was treated under vacuum at 150 °C for 3 hours. The analysis conditions were as follows.

### [Measurement Conditions]

Measurement instrument: High-speed specific surface area / pore size distribution analyzer, BELSORP-mini II (manufactured by MicrotracBEL)
Adsorption gas: Nitrogen, 99.99995 vol%
Adsorption temperature: Liquid-nitrogen temperature (-196 °C)

The compound of the present invention can be used as a catalyst for various chemical reactions. Since the catalytic activity of the compound of the present invention does not become deactivated in the presence of water molecules, it can be used particularly as a catalyst for promoting various chemical reactions in aqueous solutions. Specific examples of such chemical reactions include various dehydration reactions, allylation reactions, aldol condensation reactions, Michael addition reactions, alkylation reactions, isomerization reactions, and hydrolysis reactions. Among these, preferred examples of the chemical reactions include skeletal isomerization reactions of saccharides and dehydration reactions of saccharides. More specifically, reactions for producing HMF from saccharides can be mentioned.

The saccharides may each be in the D-form, the L-form, or a mixture thereof. In the mixture, the content of the D-form is preferably 20% by mass or more and less than 100% by mass, more preferably 90% by mass or more and less than 100% by mass, even more preferably 99% by mass or more and less than 100% by mass, and particularly preferably 99.9% by mass or more and less than 100% by mass, relative to the total mass of the D-form and L-form.

Specific examples of the saccharides include allose, talose, gulose, glucose, altrose, mannose, galactose, idose, psicose, fructose, sorbose, and tagatose, among which glucose and fructose are preferred. These saccharides may be used alone or in combination of two or more thereof.

When the compound of the present invention is used as a catalyst for various chemical reactions, there is no particular limitation on the form of the compound, but it is preferably in a particulate form. When a chemical reaction using the compound of the present invention is carried out in a batch system, the particle diameter of the compound of the present invention, as measured by X-ray diffraction (XRD) or small-angle X-ray scattering (SAXS), is preferably in the range of 1 nm to 250 nm, more preferably in the range of 3 to 50 nm, and even more preferably in the range of 5 to 10 nm.

When a chemical reaction using the compound of the present invention is carried out in a flow system, the lower limit value of the volume-average particle diameter of the compound of the present invention can be appropriately selected depending on the chemical reaction. From the viewpoint of reducing the back pressure of a column packed with catalyst particles, the lower limit value of the volume-average particle diameter of the compound of the present invention is preferably 1 µm or more, more preferably 5 µm or more, even more preferably 10 µm or more, still more preferably 20 µm or more, further preferably 30 µm or more, and most preferably 40 µm or more.

The upper limit value of the volume-average particle diameter is not particularly limited, and may be appropriately selected depending on the chemical reaction. From the viewpoint of increasing the surface area per unit volume, the upper limit value of the volume-average particle diameter is preferably 500 µm or less, more preferably 200 µm or less, and even more preferably 100 µm or less. When a chemical reaction using the compound of the present invention is carried out in a flow system, the compound of the present invention may be in the form of particles obtained by granulating particles used in batch-type reactions to a required volume-average particle diameter by a granulation method such as extrusion molding and tumbling granulation.

The production of HMF according to the present invention comprises a step of heating an aqueous saccharide solution in the presence of the compound of the present invention. Hereinafter, the aqueous saccharide solution may be referred to as "the present reaction solution," and a mixture obtained by adding the compound of the present invention to the present reaction solution may be referred to as "the present mixture." The method for producing HMF may be carried out by either a batch-type process or a flow-type process.

When the method for producing HMF is carried out in a batch-type process, the concentration of saccharides in thepresent mizture is not particularly limited; however, it is preferably in the range of 0.01 to 90 wt/vol % based on the volume of the present mixture, more preferably in the range of 1.0 to 85 wt/vol %, even more preferably in the range of 2.0 to 80 wt/vol %, and particularly preferably in the range of 3.0 to 70 wt/vol %.

When the method for producing HMF is carried out in a flow-type process, the concentration of saccharides in the present reaction solution is preferably in the range of 0.5 to 50 wt%, more preferably in the range of 1 to 40 wt%, and even more preferably in the range of 5 to 30 wt%.

When the method for producing HMF is carried out in a batch-type process, the concentration of the compound in the present mixture is not particularly limited, but is preferably in the range of 0.05 to 300 wt% relative to the saccharides, and more preferably in the range of 1 to 200 wt%.

When the method for producing HMF is carried out in a batch-type process or a flow-type process, the reaction temperature (that is, the temperature at which the present reaction solution is brought into contact with the compound; the same applies hereinafter) is preferably in the range of 80 to 180 °C, and more preferably in the range of 100 to 170 °C.

When the method for producing HMF is carried out in a batch-type process, the reaction time is a period during which the conversion of glucose or fructose and the yield of HMF reach desired values, and is appropriately selected depending on conditions such as the reaction temperature, but is preferably in the range of 10 minutes to 24 hours, and more preferably in the range of 2 to 6 hours.

When the method for producing HMF is carried out in a flow system, the reaction time is preferably in the range of 1 minute to 2 hours, and more preferably in the range of 5 minutes to 30 minutes.

It should be noted that, when carrying out the above-described method for producing HMF, whether adopting a batch system or a flow system, any means capable of performing reactions under high temperature and high pressure conditions may be employed as appropriate.

Further, the method A for producing HMF according to the present invention comprises a step of heating a saccharide aqueous solution A containing dimethyl sulfoxide and/or a polar protic organic solvent in the presence of the compound of the present invention or a compound in which a phosphono group (-PO₃H₂) is covalently bonded to a titanium oxide (hereinafter sometimes referred toherein as "phosphonated titanium oxide"). Hereinafter, the compound of the present invention and the phosphonated titanium oxide are collectively referred to as "specific compounds."

Hereinafter, in the method A for producing HMF according to the present invention, the saccharide aqueous solution A containing dimethyl sulfoxide and/or a polar protic organic solvent is also referred to as a "specific reaction solution," and the mixture obtained by adding the specific compounds to the specific reaction solution is also referred to as a "specific mixture." In this specification, the specific reaction solution and the specific mixture may be separated into a water phase and an oil phase. The method for producing HMF can be carried out in either a batch-type process or a flow-type process.

The phosphonated titanium oxide can be produced by using phosphoric acid in place of sulfuric acid in the method for producing the compound of the present invention. The phosphonated titanium oxide also has properties similar to those of the compound of the present invention, and details thereof are the same as those described above for the compound of the present invention.

In the phosphonated titanium oxide, whether a phosphono group is covalently bonded to the titanium oxide can be confirmed in accordance with the description in International Publication No. WO 2012/108472.

When the method A for producing HMF is carried out in a batch-type process, the concentration of dimethyl sulfoxide in the specific mixture is preferably in the range of 0.1 to 1.0 M, more preferably in the range of 0.2 to 0.8 M, even more preferably in the range of 0.3 to 0.7 M, and particularly preferably in the range of 0.4 to 0.6 M.

When the HMF production method A is carried out in a flow-type process, the concentration of dimethyl sulfoxide in the specific reaction solution is preferably in the range of 0.1 to 1.0 M, more preferably in the range of 0.2 to 0.8 M, even more preferably in the range of 0.3 to 0.7 M, and particularly preferably in the range of 0.4 to 0.6 M.

In the case where the HMF production method A is carried out in a batch mode, the content of the polar protic organic solvent in the specific mixture is preferably in a range of 20 to 200 vol% relative to the total volume of the specific mixturen, more preferably in a range of 50 to 150 vol%, even more preferably in a range of 70 to 130 vol%, and particularly preferably in a range of 80 to 120 vol%.

In the case where the HMF production method A is carried out in a flow mode, the content of the polar protic organic solvent in the specific reaction solution is preferably in a range of 20 to 200 vol% relative to the total volume of the specific reaction solution, more preferably in a range of 50 to 150 vol%, even more preferably in a range of 70 to 130 vol%, and particularly preferably in a range of 80 to 120 vol%.

As the polar protic organic solvent contained in the specific reaction solution or the specific mixture, examples include alcohols, aliphatic amines, alicyclic amines, acid amides, and carboxylic acids. These may be used alone or in combination of two or more thereof.

The polar protic organic solvent is preferably an alcohol having 1 to 6 carbon atoms. Specific examples of alcohols having 1 to 6 carbon atoms include methanol, ethanol, n-propanol, isopropanol, cyclopropanol, n-butanol, isobutanol, s-butanol, cyclobutanol, n-pentanol, isopentanol, s-pentanol, neopentyl alcohol, cyclopentanol, n-hexanol, isohexanol, s-hexanol, 2,2-dimethyl-1-butanol, and cyclohexanol. Among these, alcohols having 3 to 5 carbon atoms are preferred. These may be used alone or in combination of two or more thereof.

Dimethyl sulfoxide and the polar protic organic solvent may each be used alone, but it is more preferable to use both in combination.

The specific mixture or the specific reaction solution may contain, or may not contain, components other than water, the saccharides, the compound of the present invention, the phosphonated titanium oxide, dimethyl sulfoxide, and the polar protic organic solvent (hereinafter also referred to as "other components"). The content of the other components is preferably in the range of 0-200 mass% relative to the total mass of the specific mixture or the specific reaction solution, more preferably in the range of 0-20 mass%, even more preferably in the range of 0-5 mass%, and particularly preferably in the range of 0-1 mass%.

In the case where the HMF production method A is carried out in a batch mode, the concentration of the saccharides in the specific mixture is not particularly limited, but is preferably in the range of 0.01-90 mass/volume (wt/v) % relative to the volume of the specific mixture, more preferably in the range of 1.0-85 mass/volume %, even more preferably in the range of 2.0-80 mass/volume %, and particularly preferably in the range of 3.0-70 mass/volume %.

In the case where the HMF production method A is carried out in a flow mode, the concentration of the saccharides in the specific reaction solution is preferably in the range of 0.5-50 mass %, more preferably in the range of 1-40 mass %, and even more preferably in the range of 5-30 mass %.

In the case where the HMF production method A is carried out in a batch mode, the concentration of the specific compound in the specific mixture is not particularly limited but is preferably in the range of 0.05-300 mass % relative to the saccharides, and more preferably in the range of 1-200 mass %. It should be noted that the specific compound is either the compound of the present invention alone, the phosphorylated titanium oxide alone, or a mixture of the compound of the present invention and the phosphorylated titanium oxide in an appropriate ratio.

In the case where the HMF production method A is carried out in a batch mode or a flow mode, the reaction temperature is preferably in the range of 80-180°C, and more preferably in the range of 100-170°C.

In the case where the HMF production method A is carried out in a batch mode, the reaction time is a period during which the conversion rate of saccharides and the yield of HMF reach desired values and is appropriately selected depending on conditions such as the reaction temperature; however, it is preferably in the range of 10 minutes to 24 hours, and more preferably in the range of 2 hours to 6 hours.

In the case where the HMF production method A is carried out in a flow mode, the reaction time is preferably in the range of 1 minute to 2 hours, and more preferably in the range of 5 minutes to 30 minutes.

It should be noted that, in carrying out the HMF production method A, whether a batch mode or a flow mode is adopted, the reaction vessel may be selected as appropriate to be a vessel capable of performing reactions under high-temperature and high-pressure conditions.

Further, the HMF production method B of the present invention comprises a step of heating a saccharide aqueous solution B by irradiating with microwaves in the presence of the compound of the present invention or the phosphorylated titanium oxide.

Specific examples of a chemical reaction apparatus for carrying out a chemical reaction by irradiating with microwaves include those disclosed in JP-A-2011-235262, JP-A-2011-235263, JP-A-2011-240213, or the like.

The frequency of the microwaves used for irradiation can be appropriately selected depending on the reaction temperature and the reaction time to be set. Specifically, the frequency is preferably in the range of 300 MHz to 300 GHz, more preferably in the range of 900 MHz to 50 GHz, and more specifically includes 2.45 GHz, 5.8 GHz, 24 GHz, and 913 MHz. Further, heating may be carried out by irradiating microwaves having two or more frequencies.

The irradiation output of the microwaves can be appropriately determined depending on the type of reaction, the scale, or the like. The microwave irradiation method may be a multimode type or a single-mode type. In addition, the microwave irradiation may be carried out continuously or intermittently (discontinuously)

The irradiation time of the microwaves is preferably 10-100% relative to the total reaction time, more preferably 50-100%, and even more preferably 80-100%. Further, in the method B for producing HMF, the reaction time is, for example, preferably in the range of 1 minute to 2 hours, and more preferably in the range of 5 minutes to 30 minutes.

In the method B for producing HMF, the reaction vessel may be selected as appropriate to be a vessel capable of carrying out reactions under high temperature and high pressure. The output of the microwave may be appropriately adjusted so as to achieve a desired reaction temperature. In addition, in the method B for producing HMF, the heat source for the reaction may consist only of microwaves, or other heat sources may be used in combination.

In the method B for producing HMF, the aqueous sugar solution B preferably contains dimethyl sulfoxide and/or a polar protic organic solvent. The amounts of dimethyl sulfoxide and the polar protic organic solvent to be used, and specific examples of the polar protic organic solvent, are as described above.

The method B for producing HMF can be carried out by either a batch method or a flow method. In each of the methods B for producing HMF, the sugars are, specifically, as described above, and among them, glucose and fructose are preferred. These may be used alone or in combination of two or more thereof.

In the method for producing HMF of the present invention, as well as in the methods A and B for producing HMF of the present invention, glucose is subject to skeletal isomerization into fructose by the compound of the present invention, and is further converted into HMF by sequential dehydration reactions. Since the compound of the present invention functions without deactivation even in an aqueous solvent, it can be used as a catalyst for chemical reactions in an aqueous solution.

It is presumed that, by imparting sulfonic acid groups to the titanium oxide, the compound of the present invention acquires a Brønsted acid role, whereby the dehydration reaction is promoted even without the use of DMSO or the polar protic organic solvent, resulting in favorable reactivity. In addition, in the method for producing HMF of the present invention, as well as in the methods A and B for producing HMF of the present invention, it is presumed that the use of DMSO or the polar protic organic solvent makes it possible to lower the activation energy of the dehydration reaction, thereby further facilitating the progress of the reaction.

Next, the present invention will be described in greater detail using Examples; however, the scope of the present invention is not limited thereto.

### (High-Performance Liquid Chromatography (HPLC) Measurement Method)

Analysis was performed under the following conditions using a photodiode array (PDA; 254 nm and 283 nm) detector and a refractive index (RI) detector.
HPLC system: LC-2000 (JASCO Corporation)
Column: Aminex HPX-87H column (7.8 mm inner diameter × 300 mm length, Bio-Rad Laboratories, Inc.)
Eluent: 0.5 mM H₂SO₄ (prepared by 100-fold dilution of a 0.5 mol/L sulfuric acid (1 N) reagent solution; Kanto Chemical Co., Inc., Cat. No. 37880-08)
Flow rate: 0.5 mL/min
Column temperature: 308 K
The sample to be measured was diluted with water and injected in an amount of 10 µL. The retention time of HMF was 41.7 minutes. Quantification was carried out using a calibration curve method.

Unless otherwise specified with respect to temperature, all treatments and processes in each test described herein were carried out at 25 °C.

### [Example 1]

Anatase-type titanium oxide (36 g; product name: ST-01, manufactured by Ishihara Sangyo Kaisha, Ltd.; X-ray particle size: 7 nm) was added to 1,800 mL of a 1 M aqueous sulfuric acid solution, and the mixture was stirred for 48 hours. The reaction solution was filtered, and the resulting residue was washed with distilled water until the pH of the filtrate reached 3 to 4. The obtained white solid was dried at 120 °C for 16 hours to yield 32 g of a compound in which sulfonic acid groups are covalently bonded to titanium oxide, in the form of a white solid (the above pH refers to the pH at 25 °C). The BET specific surface area of the obtained compound was measured to be 280 m²/g.

Measurement of the FT-IR spectrum revealed that, whereas no peak was observed in the vicinity of 1180 cm⁻¹ for the titanium oxide that had not been subjected to sulfuric acid treatment, the compound of the present invention exhibited a signal (peak) near 1180 cm⁻¹ attributable to the stretching vibration of an S-O bond. This result indicates that a sulfonic acid group is covalently bonded to hydroxyl groups (Ti-OH) on the framework surface of the titanium oxide, thereby forming a chemical structure of Ti-O-SO₂(OH).

### [Reference Example]

A mixture comprising 1800 mL of a 1 M aqueous phosphoric acid solution, 18 mL of nitric acid (specific gravity: 1.38), and 36 g of anatase-type titanium oxide (product name: ST-01, manufactured by Ishihara Sangyo Kaisha, Ltd.; X-ray particle size: 7 nm) was stirred for 48 hours. The reaction solution was then filtered, and the residue was washed with distilled water until the pH of the filtrate reached 3-4. The resulting white solid was dried at 120 °C for 16 hours to obtain 32 g of a solid Lewis acid in which a phosphono group is covalently bonded to the titanium oxide. The BET surface area of the obtained solid Lewis acid was measured to be 290 m²/g.

### [Example 2]

A mixed solution was prepared by combining 1.0 g of the compound obtained in Example 1, 0.5 g of D-glucose and distilled water to obtain a total volume of 5 mL. The resulting mixed solution was placed in a pressure-resistant glass vessel and sealed. The sealed vessel was then maintained in a closed state, and the mixed solution was heated at 120 °C for 4 hours with stirring to carry out the reaction. Quantitative analysis of the resulting reaction solution by high-performance liquid chromatography confirmed that HMF was obtained at a concentration of 94 mM in the reaction solution. Prior to HPLC analysis, the compound obtained in Example 1 was removed from the reaction solution by filtration. Accordingly, the reported HMF concentration represents the concentration of HMF in the reaction solution after removal of the compound obtained in Example 1. The same applies to the following test examples. In the solution of this Example, the amount of the compound obtained in Example 1 corresponds to 200 mass% relative to the total mass of D-glucose, and the amount of D-glucose corresponds to 10 mass/volume% relative to the volume of the mixed solution.

### [Example 3]

In the mixed solution of Example 2, dimethyl sulfoxide (DMSO) was further added in an amount of 2.2 mmol, corresponding to a DMSO concentration of 0.43 M in the resulting solution to which DMSO was added. Except for this addition, the procedure was carried out in the same manner as in Example 2. As a result, it was confirmed that HMF was obtained at a concentration of 108 mM in the reaction solution.

### [Example 4]

In the mixed solution of Example 2, 5 mL of n-butanol was further added. Except for this addition, the procedure was carried out in the same manner as in Example 2. As a result, HMF was obtained at a concentration of 148 mM. It should be noted that the reaction solution was separated into a water phase and an oil phase. The HMF concentration described herein represents the concentration calculated for the reaction solution as a whole, that is, the average concentration of HMF across both the aqueous phase and the organic phase. The same definition applies hereinafter to cases in which the reaction solution is separated into an aqueous phase and an organic phase.

### [Example 5]

Except that the amount of added D-glucose was changed to 1.25 g, the procedure was carried out in the same manner as in Example 4, and HMF was obtained at a concentration of 271 mM in the reaction solution.

### [Example 6]

Except that the amount of added D-glucose was changed to 2.0 g, the procedure was carried out in the same manner as in Example 4, and HMF was obtained at a concentration of 382 mM in the reaction solution.

### [Example 7]

Except that 2.2 mmol of dimethyl sulfoxide (DMSO) and 5 mL of n-butanol were further added to the mixed solution in Example 2, the procedure was carried out in the same manner as in Example 2, and HMF was obtained at a concentration of 150.1 mM in the reaction solution.

### [Example 8]

Except that the amount of added D-glucose was changed to 1.25 g, the procedure was carried out in the same manner as in Example 7, and HMF was obtained at a concentration of 285.5 mM in the reaction solution.

### [Example 9]

Except that the amount of added D-glucose was changed to 2.0 g, the procedure was carried out in the same manner as in Example 7, and HMF was obtained at a concentration of 390.4 mM in the reaction solution.

### [Example 10]

A cylindrical pressure-resistant column (inner diameter: 7.6 mm, height: 150 mm) was packed with the compound obtained in Example 1 and maintained at 160 °C. Through this cylindrical pressure-resistant column kept at 160 °C, a 25 wt% aqueous glucose solution (reaction solution) was passed at a flow rate of 0.5 mL/min for 4 hours. The number of passes was one. Quantification of the reaction solution after passage by high-performance liquid chromatography confirmed that HMF was obtained at a concentration of 125 mM in the reaction solution. The average contact time between the reaction solution and the compound of the present invention in this example was 13.6 minutes.

### [Example 11]

A mixture was prepared by combining 1.0 g of the compound obtained in Example 1, 2.0 g of D-glucose, and distilled water to make a total volume of 5 mL. The resulting mixture was placed in a pressure-resistant glass vessel and sealed. While maintaining the vessel in a sealed state, the mixture inside was stirred and irradiated with microwaves having a frequency of 2.45-GHz for 10 minutes. The microwave power was adjusted such that the temperature of the mixture during irradiation reached 160 °C. Quantification of the resulting reaction solution by high-performance liquid chromatography confirmed that HMF was obtained at a concentration of 271 mM in the reaction solution. In this example, the amount of the compound of the present invention in the mixture corresponded to 50 wt% relative to the total mass of D-glucose, and the amount of D-glucose corresponded to 40 wt/vol% relative to the total volume of the mixture.

### [Example 12]

Except that 2.2 mmol of dimethyl sulfoxide (DMSO) was additionally added to the mixture used in Example 11 (corresponding to a DMSO concentration of 0.43 M in the mixture), the procedure was carried out in the same manner as in Example 11, and HMF was obtained at a concentration of 392 mM in the reaction solution.

### [Example 13]

Except that 5 mL of n-butanol was additionally added to the mixture used in Example 11, the procedure was carried out in the same manner as in Example 11, and HMF was obtained at a concentration of 485 mM in the reaction solution.

### [Example 14]

Except that the amount of D-glucose added was changed to 0.5 g and 5 mL of n-butanol was additionally added, the procedure was carried out in the same manner as in Example 11, and HMF was obtained at a concentration of 170 mM in the reaction solution.

### [Example 15]

Except that the amount of D-glucose added was changed to 1.25 g and 5 mL of n-butanol was additionally added, the procedure was carried out in the same manner as in Example 11, and HMF was obtained a concentration of 322 mM in the reaction solution.

### [Example 16]

A solution was prepared by mixing 1 g of the compound obtained in the Reference Example, 0.5 g of D-glucose, and distilled water to make a total volume of 5 mL, and then adding 5 mL of n-butanol. The resulting mixed solution was placed in a pressure-resistant glass vessel and sealed. The mixture was stirred and reacted at 120°C for 4 hours. The reaction solution was quantified by high-performance liquid chromatography, and HMF was obtained at a concentration of 148.8 mM in the reaction solution.

### [Example 17]

Except that 2.2 mmol of dimethyl sulfoxide (DMSO) was further added to the mixed solution of Example 16, the procedure was carried out in the same manner as in Example 16. As a result, HMF was obtained at a concentration of 180.9 mM in the reaction solution.

### [Example 18]

Using the compound obtained in the Reference Example, and except that DMSO was added to the 25% aqueous glucose solution so that the concentration of DMSO in the entire solution after addition became 0.43 M, the procedure was carried out in the same manner as in Example 10. As a result, HMF was obtained at a concentration of 151 mM in the reaction solution.

### [Example 19]

Except that D-fructose was used instead of D-glucose, the procedure was carried out in the same manner as in Example 2, and HMF was obtained at a concentration of 154.2 mM in the reaction solution.

### [Comparative Example 1]

Except that the compound obtained in the Reference Example was used, the procedure was carried out in the same manner as in Example 10, and HMF was obtained at a concentration of 113 mM in the reaction solution.

### [Comparative Example 2]

Except that D-fructose was used instead of D-glucose and anatase-type titanium oxide (product name: ST-01, manufactured by Ishihara Sangyo Kaisha, Ltd.; X-ray particle size: 7 nm) was used instead of the compound obtained in Example 1, the procedure was carried out in the same manner as in Example 2, and HMF was obtained at a concentration of 89.8 mM in the reaction solution.

### [Comparative Example 3]

Except that anatase-type titanium oxide (product name: ST-01, manufactured by Ishihara Sangyo Kaisha, Ltd.; X-ray particle size: 7 nm) was used instead of the compound obtained in Example 1, the procedure was carried out in the same manner as in Example 4, and HMF was obtained at a concentration of 55.3 mM in the reaction solution.

### [Comparative Example 4]

Except that tetrahydrofuran was used instead of n-butanol and the mixture was heated at 70 °C, the procedure was carried out in the same manner as in Example 4, and HMF was obtained at a concentration of 0.5 mM in the reaction solution.

### [Industrial Applicability]

The method for producing HMF of the present invention is useful as an industrial process for producing HMF from sugars such as glucose.

## Claims

1. A compound having a sulfonic acid group covalently bound to a titanium oxide.

2. The compound according to Claim 1, wherein the BET specific surface area is 200 m²/g or more.

3. The compound according to Claim 1 or 2, wherein the BET specific surface area is 300 m²/g or less.

4. The compound according to Claim 1 or 2, wherein the compound is a solid Lewis acid.

5. The compound according to Claim 1 or 2, wherein the compound is a catalyst for a chemical reaction in an aqueous solution.

6. The compound according to Claim 5, wherein the chemical reaction is a dehydration reaction of a saccharide.

7. The compound according to Claim 5, wherein the chemical reaction is a reaction that produces 5-hydroxymethylfurfural from a saccharide.

8. The compound according to Claim 6, wherein the saccharide is glucose and/or fructose.

9. The compound according to Claim 7, wherein the saccharide is glucose and/or fructose.

10. A method for producing 5-hydroxymethylfurfural, comprising heating an aqueous saccharide solution in the presence of the compound according to Claim 1 or 2.

11. A method for producing 5-hydroxymethylfurfural, comprising heating an aqueous saccharide solution containing dimethyl sulfoxide and/or a polar protic organic solvent in the presence of the compound according to Claim 1 or 2, or a compound having a phosphono group covalently bounded to titanium oxide.

12. A method for producing 5-hydroxymethylfurfural, comprising irradiating an aqueous saccharide solution with microwaves to heat it in the presence of the compound according to Claim 1 or 2, or a compound having a phosphono group covalently bounded to titanium oxide.

13. The method for producing 5-hydroxymethylfurfural according to Claim 12, wherein the aqueous saccharide solution is an aqueous solution containing dimethyl sulfoxide and/or a polar protic organic solvent.

14. The method for producing 5-hydroxymethylfurfural according to Claim 11, wherein the polar protic organic solvent is an alcohol having 1 to 6 carbon atoms.

15. The method for producing 5-hydroxymethylfurfural according to Claim 13, wherein the polar protic organic solvent is an alcohol having 1 to 6 carbon atoms.

16. The method for producing 5-hydroxymethylfurfural according to Claim 11, wherein the polar protic organic solvent is an alcohol having 3 to 5 carbon atoms.

17. The method for producing 5-hydroxymethylfurfural according to Claim 13, wherein the polar protic organic solvent is an alcohol having 3 to 5 carbon atoms.

18. The method for producing 5-hydroxymethylfurfural according to Claim 10, wherein the saccharide is glucose and/or fructose.

19. The method for producing 5-hydroxymethylfurfural according to Claim 11, wherein the saccharide is glucose and/or fructose.

20. The method for producing 5-hydroxymethylfurfural according to Claim 12, wherein the saccharide is glucose and/or fructose.

21. The method for producing 5-hydroxymethylfurfural according to Claim 11, wherein the compound having a phosphono group is covalently bonded to a titanium oxide is a solid Lewis acid.

22. The method for producing 5-hydroxymethylfurfural according to Claim 12, wherein the compound having a phosphono group covalently bonded to titanium oxide is a solid Lewis acid.

23. A method for producing the compound according to Claim 1 or 2, comprising treating a titanium oxide with sulfuric acid.
